# EUROPEAN PATENT SPECIFICATION

(11) **EP 2 334 298 B1**
(45) Date of publication and mention of the grant of the patent: **30.07.2014**
(21) Application number: 09741449.4
(22) Date of filing: 15.09.2009
(51) Int. Cl.: A61K 31/43, A61K 31/7036, A61P 31/04

(54) **NOVEL SINGLE UNIT CARBAPENEM AMINOGLYCOSIDE FORMULATIONS**
NEUE EINZELNE CARBAPENEM-AMINOGLYKOSID-FORMULIERUNGEN
NOUVELLES FORMULATIONS DE CARBAPENEM ET D'AMINOGLYCOSIDE EN UNE SEULE UNITÉ

(30) Priority: 18.09.2008 IN DE21842008
(43) Date of publication of application: 22.06.2011
(73) Proprietor: Chaudhary, Manu, Panchkula 134 113 Haryana (IN)
(72) Inventor: Chaudhary, Manu, Panchkula 134 113 Haryana (IN)
(74) Representative: Heinze, Ekkehard
(86) International application number: PCT/IN2009/000508
(87) International publication number: WO 2010/032266

(56) References cited:
- WO-A1-2006/064516
- WO-A2-2006/120705
- HOSGOR-LIMONCU M ET AL: "Activity of amikacin, ertapenem, ciprofloxacin and levofloxacin alone and in combination against resistant nosocomial pathogens by time-kill" WEST INDIAN MEDICAL JOURNAL 200803 JM, vol. 57, no. 2, March 2008 (2008-03), pages 106-111, XP9132491 ISSN: 0043-3144
- LATZIN ET AL: "Efficacy and safety of intravenous meropenem and tobramycin versus ceftazidime and tobramycin in cystic fibrosis" JOURNAL OF CYSTIC FIBROSIS, ELSEVIER LNKD- DOI:10.1016/J.JCF.2007.07.001, vol. 7, no. 2, 1 March 2008 (2008-03-01), pages 142-146, XP022524293 ISSN: 1569-1993
- MATHE ET AL: "The effect of amikacin and imipenem alone and in combination against an extended-spectrum beta-lactamase-producing Klebsiella pneumoniae strain" DIAGNOSTIC MICROBIOLOGY AND INFECTIOUS DISEASES, ELSEVIER SCIENCE PUBLISHING CO., AMSTERDAM, NL LNKD- DOI:10.1016/J.DIAGMICROBIO.2006.11.013, vol. 58, no. 1, 10 May 2007 (2007-05-10), pages 105-110, XP022068555 ISSN: 0732-8893
- MIRANDA-NOVALES GUADALUPE ET AL: "In vitro activity effects of combinations of cephalothin, dicloxacillin, imipenem, vancomycin and amikacin against methicillin-resistant Staphylococcus spp. strains", ANNALS OF CLINICAL MICROBIOLOGY AND ANTIMICROBIALS, BIOMED CENTRAL, LPNDON, GB, vol. 5, no. 1, 12 October 2006 (2006-10-12), page 25, XP021024305, ISSN: 1476-0711, DOI: 10.1186/1476-0711-5-25

## Description

### FIELD OF INVENTION:

The field of invention pertains to pharmaceutical formulations. More specifically, it pertains to low dose synergistic formulations of two antibiotics selected from two different groups viz. a carbapenem and an **aminoglycoside present as a single unit fixed low dose injection, for the treatment of bacterial / multi bacterial infections. Invention further pertains to formulations which can overcome** Carbapenem and aminoglycoside resistance and which lower drug and disease induced toxicity making it ideal for use in neonates, children and other patients with low tolerance who are prone to serious fatal infectious diseases.

### BACKGROUND OF INVENTION:

Antibiotic resistance has become a serious public health concern with economic and social implications throughout the world, be it community acquired infections like Streptococcal infections, pneumonia, typhoid fever, etc.,or hospital acquired infections due to methicillin resistant Staphylococcus aureus (MRSA), vancomycin resistant enterococci (VRE), vancomycin intermediate S. aureus (VISA). There have been increased incidence of bacterial resistance to antibiotics in the past 15 years, in spite of the introduction of potent new antibacterial agents belonging to novel chemical classes such as penems, cephems, oxacephems, monobactams.

Carbepenems are still the drug of choice for multi-drug resistant organisms. [Rahal JJ Critical Care 2008, 12(Suppl 4):S5]. This group of antibiotics is rapidly replacing cephalosporins and quinolones in the treatment of multi-drug resistant Gram negative bacteria and are also used to treat nosocomial and mixed bacterial infections. However, nosocomial isolates may easily develop resistance to carbapenems due to the reduced uptake of the drug, which leads to outbreaks of imipenem/ meropenem resistant strains. [El Amin N. et al, APMIS 2005;113:187-96.]

Shashikala et al. [Indian J Pharmacol 2006, 38 (4),287-288] showed the high prevalence of carbapenem resistance among *P. aeruginosa* strains isolated from relevant clinical specimens. Another study by [Taneja et al, Indian J Med Sci 2003;57:294-9]showed 42% resistance to imipenem in urinary isolates.

Though the Carbapenems are used to treat serious infections, as the last resort, when the organism is resistant to the primary agents of choice still the emerging resistance to carbapenems further limits therapeutic options.

At the same time combinations of multiple antibiotics have been conventionally investigated aiming at the enhancement of antibacterial potency such as Nakamura et al [Journal of Antimicrobial Chemotherapy. 2000 ; (46) :901-04] have reported that combination of Meropenem with each of three aminoglycosides, arbekacin , amikacin and netlimicin were effective against almost all *P. aeruginosa* strains but these strains were susceptible to meropenem also.

Mueller et al [Critical Care 2003, 7(Suppl 2):P126] reported that Carbapenems such as imipenem shows important antimicrobial activities against *Pseudomonas aeruginosa* and *Acinetobacter calcoaceticus*/*baumanii* when additionally coadministration with Gentamicin and/or Amikacin aminoglycoside.

Katou et al [Chemotherapy 2005;51:387-391]have studied that combinations of Panipenem and aminoglycosides (arbekacin, amikacin , vancomycin and netlimicin) showed additive effects against MRSA and *P. aeruginosa.*

Tasaka et al [Jpn J Antibiot.2002 :55(2):181-6]suggested that meropenem is superior to imipenem in combined effect with amikacin against P. aeruginosa. Further meropenem showed higher antipseudomonal activities than other carbapenems tested in both conditions, alone and in combination with amikacin. With regard to the clinical efficacy and prevention of antibiotic resistance, meropenem mono therapy or combination therapy with aminoglycoside is the most superior treatment for pseudomonal infections which are susceptible to penems.

Besides the problem of resistance, carbapenems also show high dose related toxicities. Traditional therapies with carbapenems also have significant side effects such as Norrby et al.(Drug Safety. 1996 :15(2): 87-90) discussed that like other betalactam antibacterials, carbapenems have a neurotoxic potential that seems to be higher than that of the penicillins and cephalosporins. Seizures have been reported in several large studies of patients treated with high doses of imipenem/cilastatin.

Other problametic side effects include nephrotoxicity and seizures associated with administration of carbapenems. Bruce M. Tune [Pediatr Nephrol (1997) 11: 768-772] discussed the significant renal toxicity, which has been rare with the penicillins and uncommon with the cephalosporins, is a greater risk with the penems.

Arnold H Seto [The Annals of Pharmacotherapy,2005: Vol. 39, No. 2, pp. 352-356] described Ertapenem-associated Seizures in a Peritoneal Dialysis Patient. These side effects often discourage medical fraternity from following the recommended therapeutic regimen. According to the World Health Organization, S. pneumoniae is the leading cause of severe pneumonia worldwide in children younger than 5 years old, causing more than 1 million deaths in children each year [Pneumococcal Vaccines: WHO Position Paper: Wkly Epidemiol Rec, Vol 74, 177-183, 1999]. Currently accepted therapy for severe bacterial respiratory tract infections, particularly for treatment of pneumonia in patients with underlying illnesses, includes treatment with various intravenous antibacterial agents. These are often used in two or three way combination.

CNS infections are a significant cause of morbidity and mortality in children. Even with antibiotic therapy, bacterial meningitis remains a serious cause of infant morbidity and mortality and represents a bacteriological emergency requiring urgent diagnosis and treatment. The World Health Organization estimates that bacterial meningitis strikes 426,000 children younger than 5 years annually, with 85,000 deaths. The incidence of neonatal meningitis has shown no significant change in the last 25 years despite significant advances in scientific knowledge and development of new antibiotics.

U. S. Patent No. 4,757,066 discloses a composition and method to eliminates the renal problems associated with administration of the carbapenem antibiotic when administred with N-acylated aminoacid. Another U. S. Patent applicatio No. 2005/0020567A1 describes a method of treating anti-bacterial infections using gemifloxacin (flouroquinolone) and carbapenem antibiotic where the separate, simultaneous or sequential administration is done to achieve the efficacy. U.S. Patent No. 6,221,859 B1 describes the use of novel 2-(naphthosultamyl)methyl-carbapenem antibacterial agents in combination with other .beta.-lactams, which are useful in treating and preventingenterococcal infections. But none of prior arts in patent/research publications disclosed about a single fixed dose formulation containing carbepenm and aminoglycoside. Further they fail to disclose a formulation which along with additives agents canovecome carbapenem and aminoglycoside resistance simultaneously reducing drug and disease induced toxicity.

In the light of above discussion and references quoted in background of this application it is very much clear that resistance among Carbapenems is increasing at an alarming rate, leading to greater patient morbidity and mortality from Hospital acquired infections as well as Community acquired infections. Further high dose related toxicities is also a major concern for the safe use of carbapenems. Therefore there is an ever pressing need of protection and enhancement of bacteriostatic /bactericidal activity of existing antibiotics and to reduce high dose related toxicities of existing carbapenems and other antibiotics. Hence, there was a related toxicities of existing carbapenems and other antibiotics. Hence, there was a great need of the product of current invention which can take care of overcoming drug reistance, reducing drug and disease induced toxicities, besides increasing efficacy that too at very low drug concentartions and is avialble as single unit for injection.

### TECHNICAL BARIER FOR CURRENT INVENTION:

Lack of suitability of existing therapy : Most of the drugs currently used lead to undesirable side-effects or even lack of efficacy due to resistance caused by prolonged administration. In case of neonates and children, where higher doses may be toxic and lower doses of existing therapies may not work. Reduction of doses to a concentration where efficacy is achieved with leeser side effects posed serious challenges.
1.Availability: The drugs which may have been found efficacious either alone or in combination may not be commercially available as a single combination formulation, owing to challenges of multiple pricks, complicated procedures and drug related incompatibility and stability.
2.Fatality : Co administration of the drugs is risky due to lack of established safety data which may result in increased fatality.
3.Toxicity and side-effects: Carbapenem/ cephalosporins/ aminoglycosides/ glycopeptides cause serious toxic and side effects as stated in the references cited above.
4. Neurotoxicity and dose limitation: ß-lactam antibiotics & penems are potentially neurotoxic and may cause seizures if given in high doses relative to renal function and/or bodyweight. Other complications include brain abscess, subdural fluid collection and focal neurologic findings. Therefore, dose limitation is a challnege in order to limit toxicity.
5. Resistance: There is a high frequency of resistance to cephalosporins and penems in Gram-negative aerobic bacilli and also an increasing prevalence of highly penicillin-resistant pneumococci.

Current invention provides a solution:
1 to overcome drug resistance
2 to reduce drug induced toxicities such as neurotoxicity, nephrotoxicity, hepatotoxicity etc.
3 To reduce disease induced oxidative stress
4 enhanced efficacy at low drug concentrations
5 safer formulation with all solutions in a single unit injection
6 stable, compatible formulations
7 broader bactericidal range with synergistic action
8 safer and better alternative for immuno compromised/ old/ neonatal patients

### SUMMARY OF THE INVENTION

The present invention provides new antibiotic combinations in which remarkable efficacy is achieved at very low concentrations. This invention further relates to novel synergistic antibiotic formulations of low concentration comprising of two different groups of antibiotics viz. a carbepenem and an aminoglycoside along with some suitable additive agents, said formulations being suitable for treatment of complicated bacterial/ multi bacterial infections. The present invention therefore provides, among other things, certain drug combinations, pharmaceutical composition formulations containing such combinations, and methods of treating patients suffering from or susceptible to mixed multi bacterial fatal infections such as respiratory, pulmonary, CNS infections and the like, especially those caused by gram-negative bacteria or bacteria resistant to antibiotics with such combinations or composition formulations by minimizing the toxic effects, side-effects, adverse effects and reduction of disease and drug induced toxicities in patients. The composition of invention has improved efficacy with dose lower than the individually established therapeutic drug concentrations of active constituents.

### OBJECTIVES:

It is an object of the invention to disclose novel synergistic antibiotic fixed dose formulations in which remarkable efficacy is achieved at very low concentrations.

Another object of the invention is to disclose fixed dose antibiotic composition and formulation made thereof which can overcome carbapenem and aminoglycoside resistance and to lower drug and disease induced toxicity.

Yet another object of the invention is to disclose safe and effective low dose antibiotic formulations which can reduce disease associated complications and minimize the risk of systemic effects.

A still further objective of the present invention is to formulate and administer a lower dose of combination with better efficacy than either of the two individually administered drugs against drug resistant bacteria. The composition improves efficacy with concentrations lower than the individually established therapeutic drug concentrations of active constituents.

A further object of the invention is to disclose new formulations of two different groups of antibiotics as active constituents, using a novel aminoglycoside and carbapenems along with additive agents present as a single unit fixed dose injections effective at very low concentration making it ideal for use in neonates , children and other patients with low tolerance which are prone to serious fatal infectious diseases.

### DETAILED DESCRIPTION:

The present invention discloses novel synergistic antibiotic fixed dose composition and formulations made thereof in which remarkable efficacy is achieved at very low concentrations with reduction in drug and disease induced toxicities. Further the invention relates to low dose combination antibiotic formulations, consisting of two different groups of antibiotics , where one of the antibiotic is any one of the carbepenems selected from a group of Meropenem, Imipenem, Ertapenem, Doripenem, panipenem or a pharmaceutically acceptable salt thereof and the other antibiotic is the aminoglycoside Etimicin or a pharmaceutically acceptable salt thereof. Further the invention leads to minimizing of toxic side-effects, adverse effects and reduction of the disease and drug induced toxicities in patients by addition of some additives to the formulations. The invention further relates to novel drug combinations, pharmaceutical compositions containing such combinations, and methods of treating patients suffering from or susceptible to mixed multi bacterial fatal infections such as pulmonary CNS infections, especially those caused by gram-negative bacteria or bacteria resistant to antibiotics with such combinations or compositions by reducing drug and disease induced toxicities.

The invention further discloses novel formulations of two antibiotics as active constituents present as single unit fixed dose formulations effective at very low drug concentrations making it ideal for use in neonates, children and other patients with low tolerance and are prone to serious fatal infectious diseases.

More specifically the invention relates to novel combinations and compositions of two entirely different groups of antibiotics where one of the antibiotic is any one of the carbepenem selected from a group detailed above , the other antibiotic is Etimicin, present as free acid or pharmaceutically acceptable salts there of such as sodium, potassium, sulphate , phosphate, hydrochloride, combined for the first time together in weight ratios of 6:1 to 13:1 was single premix unit along with some additional agents thereby enabling the composition and formulation made thereof to overcome carbapenem and aminoglycoside resistance and to lower drug and disease induced toxicity in order to provide a low dose formulations suitable for neonates, children and other patients with low tolerance which are prone to serious fatal mixed multi bacterial infectious diseases where potential toxicity due to higher doses is a cause for concern.

The combination formulations contain some additives selected from a group of synthetic/ natural amino acids/ vitamins/ stabilizers/ polymers/ antioxidants/ micro-nutrients or a a pharmaceutically acceptable salt thereof which are a part of food supplements in specific weight ratios to get additive benefits of reduction in drug and disease induced toxicities. Further these components may be either added directly in the compositions at the time of formulation or could also be added to solvent used for the reconstitution of the said formulations.

More specifically present invention relates to combination formulations comprising a novel aminoglycoside antibiotic Etimicin present as sulphate in combination with any of the Carbapenem such as Imipenem, meropenem, ertapenem or panipenem present as free acid or a pharmaceutically acceptable salts there of combined in a weight ratios of 0.076:1 to 0.166:1 with suitable additive agent selected from a group of synthetic/ natural amino acids/ vitamins/ stabilizers/ polymers/ antioxidants/ micro-nutrients or a pharmaceutically acceptable salt thereof where the weight ratio of active constituents : additives is 1: 0.0001 to 1: 0.75 thereby enabling the composition and formulation made thereof to overcome carbapenem and aminoglycoside resistance and to lower drug and disease induced toxicity. Additive agents may be selected from L arginine, EDTA, PLP, ascorbic acid, biotin, thiamine, riboflavin, polyethylamine, selenium, gluconic acid, zinc or a pharmaceutically acceptable salts thereof to get additive benefits along with the combination formulations either directly as additive to antibiotic combination formulations or as solvent to the antibiotic formulations.

It was surprisingly found that the agents which are well known such as amino acids, chelating agent, vitamins, micronutrients and the like and are also a part of food supplement are quiet helpful in reducing drug and disease induced toxicities when given in specific weight ratios along with the drug_at the time of administration which may be pre-blended with the active constituents at the time of formulation or is added to the reconstitution solution or alternatively used at the time of infusion.

Experimental results proved that conventionally well known agents such as L arginine, EDTA, PLP, ascorbic acid, biotin, thiamine, riboflavin, polyethylamine, selenium, gluconic acid, zinc or a pharmaceutically acceptable salts thereof are capable to handle the multi-organ complications in serious fatal diseases and the patients with poor tolerance and low immune power. It was further observed that drug and isease induced toxicities are usually associated with defeciency of any of the said components.

The use of amino acids to catalyze virtually all chemical reactions in the body, regulate gene expression, as the major structural elements of all cells, regulate the immune system, to form the major constituents of muscle, to serve as neurotransmitters and modulators of various physiological processes is well known. The role of L arginine as stabilizing agent has been disclosed in inventor's previous patent application No PCT/IN2005/000415. Similarly the use of EDTA as chelating agent is well known. The additional role EDTA as Particulate formation inhibitor has been discussed in inventor's previous patent application No PCT/IN2005/000382. The role of vitamins as food supplement, in management of skin disorders, nerve cell normal functioning, wound healing, formation of healthy bones and control of hormonal functions are well known.

In the current invention it was established that administration of one or more of vitamin B6 (PLP), EDTA , selenium, ployethylamine, ascorbic acid , L- arginine etc. along with antibiotics together or separately at the time of administration in specific weight ratios (active constiuents :additive agent in the ratio of 1:0.0001 to 1: 0.75) provide additional benefits in the form of reducing oxidative stress, improvement in blood flow, reduction in lipid peroxidation, reducing liver and kidney toxicities and there by help in drastically reducing disease and drug induced toxicities along with reduction in organ failure complications. One or more of these components together or alone could be either added to the formulations of antibiotics or to the solvent used for reconstitution of the formulations formed as result of invention. It was surprisingly found that these components not only reduce drug and disease induced toxic effects of the formulations of the current inventions but are equally effective for toxicity reduction of other antibiotic injectable formulations as well.

Therefore, the present invention relates to novel formulations containing two or more different groups of antibiotics as active constituents where the first antibiotics is a carbapenem selected from a group of Meropenem , Ertapenem, doripenem, imipenem or panipenem or a pharmaceutically acceptable salts thereof , second antibiotic is the aminoglycoside Etimicin, or a pharmaceutically acceptable salts thereof, present in weight ratios of carbapenem to aminoglycoside as 6:1 to 13:1 along with one or more additives selected from a group of synthetic/ natural amino acids/ vitamins/ stabilizers/ polymers/ antioxidants/ micro-nutrients or a pharmaceutically acceptable salts thereof and the like where the weight ratio of active constituents : additives is 1: 0.0001 to 1: 0.75 (as is deemed fit for the formulation) thereby enabling the composition and formulation made thereof to overcome carbapenem and aminoglycoside resistance and to lower drug and disease induced toxicity. The formulation has improved efficacy with dose lower than the individually established therapeutic drug concentrations of active constituents , is safe and is present as premix dry powder injection formulation to be reconstituted with solvent which is preferably water for injection or alternatively contains one or more of the additive agents..

One of the embodiment of the invention is to disclose a composition where the said carbapenem is meropenem or a pharmaceutically acceptable salt thereof, which is present in the range of 62.5mg to 3600mg of the said composition, the said novel aminoglycoside is etimicin or a pharmaceutically acceptable salt thereof which is etimicin sulphate present in the range of 50mg to 400 mg of the said composition along with EDTA or a pharmaceutically acceptable salt thereof where the ratio of active constituents to : EDTA is 1:0.025 to 1:0.25. The compositions additionally contain additives selected from PLP, selenium, copper, zinc or the pharmaceutically acceptable salts thereof where the ratio of active constituent: additives is 1:0.01 to 1:0.5 along with the combination formulations which are added either directly as additive to the said formulations or added in the solvent used to reconstitute the antibiotic formulations for the treatment of severe bacterial/ multi bacterial infections. The new formulations thus derived from the current invention have very low toxicity and show synergistic effect at low concentrations of drug/low doses. Owing to remarkable efficacy at low concentrations, the formulations are particularly suitable for neonates, children and other patients with low drug and disease tolerance which are prone to serious fatal mixed multi bacterial infectious diseases where potential toxicity due to higher doses is a cause for concern.

Further aminoglycoside antibiotic used in current invention is a novel antibiotic(Etimicin sulphate) which has minimal toxicity among its group. Etimicin Sulphate [(China Patent No. ZL 9311412.3), United States Patent (US 005814488A), Britain Patent (UK GB 2 293 383 B)]. Among all aminoglycoside antibiotics it has the minimal ototoxicity and nephrotoxicity. Usual dose of Etimicin sulphate injection for adults available is 100mg/1ml to 200mg/2ml to be administered every 12 hours. The drug has very good bactericidal range and broad spectrum coverage with lesser adverse effects compared to other amino glycosides. At the same time it has its own limitations. Being aminoglycoside the drug cannot be administered for longer treatments or in combination with other antibiotics with which it is incompatible.

A novel feature of the present invention is the combination of a novel aminoglycoside Etimicin and carbepenems, being disclosed for the first time.

Another novel feature of the present invention is the formulation combinations exhibit efficacy at 2.5 to 10 times lesser drug concentration than the drug concentration of conventional treatment (50%-80% dose reduction).

Another embodiment of the invention is to disclose a formulation where the said carbapenem is ertapenem or a pharmaceutically acceptable salt thereof , which is present in the range of 50 mg to 1800 mg of the said composition, or alternatively the said carbapenem is panipenem or a pharmaceutically acceptable salt thereof , which is present in the range of 25 mg to 1500 mg of the said composition, the said aminoglycoside is etimicin or a pharmaceutically acceptable salt thereof which is preferably a sulphate salt present in the range of 10mg to 450 mg of the said composition along with the said additive agent which is preferably a basic amino acid or a pharmaceutically acceptable salt thereof where the ratio of active constituents to : additive is 1:0.05 to 1:0.75. The composition optionally contains more additives such as EDTA, Selenium, zinc, PLP or a -pharmaceutically acceptable salt thereof which may be pre-blended with the active constituents at the time of formulation or is added to the reconstitution solution or alternatively used at the time of infusion.

The innovative feature of the invention is combining these two antibiotics along with one or more well defined agents like amino acids, chelating agents, vitamins and the like such as PLP (Vit B6), EDTA, selenium/ zinc and /or L arginine which are well known part of daily diet but when administered together with the antibiotic combination in specific weight ratios of 1: 0.0001 to 1:0.75 help in reducing neuro, kidney and liver toxicities by reducing oxidative stress, lipid peroxidation and improving blood flow beside overcoming the drug resistance and improved tissue penetration which ultimately results in lowering the required therapeutic drug concentrations. The said agents are either added to the formulations of antibiotics to be reconstituted with water for injection or added in solvent to the antibiotic formulation can reduce of the toxicity induced by any known antibiotic.

Yet another aspect of this invention is to disclose a significantly low-concentration antibiotic formulation for the treatment of complicated bacterial / multi bacterial infections for example those caused by *Escherichia coli, Enterobacteria species, Klebsiella pneumoniae, Proteus mirabilis, Pseudomonas aeruginosa, Streptococcus pneumoniae , MRSA, Serratia marcescens, Haemophilus influenzae, Burkholderia cepacia, Stenotrophomonas maltophilia, Alcaligenes xylosoxidans. Neisseria meningitidis (meningococcus), Haemophilus influenzae, Listeria monocytogenes, Cryptococcus neoformans.*

The method of treatment comprises parenteral administration of the formulation which is released slowly in sustained form or immediately at the site of injection and is preferably available in dry powder form after reconstitution with a suitable solvent/diluent which may be water for injection alone or any of the additives added to water for injection.

The composition is a single unit, packed in a sealed air tight pharmaceutically acceptable container which is selected from the group consisting of vial, an ampoule, a syringe, a packet, a pouch, and an autoinjecter and the like, present as dry powder for reconstitution, lyophilized powder, as a dose concentrate in the form of drug particles, powders, granules, nano-particles, microspheres and the like. Composition is suitably protected from moisture , light and degradation with the help of vacuum or inert gas micro atmosphere or in pressurized carbon di oxide.

Accordingly another aspect of the present invention is to provide pharmaceutical compositions and formulations made thereof that are safe, less toxic and have efficacy against a wide variety of infectious organisms, and to provide compositions that are useful in providing effective treatment against multi drug resistant bacteria.

A process of preparing the formulation comprises following steps:
(a) sterile filling / blending the said antibiotic ingredients or pharmaceutically acceptable salts thereof,
(b) sterile adding / blending the additives
(c) continuing said sterile adding / filling / blending for a period ranging from about 1 hour to about 6 hours,
d proportioning the sterile fill / blend, and
(e) capping aseptically with pre-post inert gassing

The process may be altered to make the formulation in lyophilized form or in sustained released micro particles/ micro-spheres using nano-technology. All such amendments lie within the scope of current invention.

It is to be understood that the invention is not limited to the particular embodiments of the invention described above, which are for limited purpose of illustrating operation of this invention, as variations of the particular embodiments obvious to a person skilled in the art may be made and still-fall within the scope of the appended claims. It is also to be understood that the terminology employed is for the purpose of describing particular embodiments, and is not intended to be limiting. Instead, the scope of the present invention will be established by the appended claims. Further, in this specification and the appended claims, the singular forms "a," "an," and "the" include reference to their plural forms too unless the context clearly dictates otherwise.

### Example 1: Efficacy at low dose (MIC DATA)

In-vitro microbial efficacy of Cilastatin+Imipenem, Meropenem, Etimicin Sulphate and Drug of Invention

| | | **MIC (mcg/ml)** | | | |
|---|---|---|---|---|---|
| **Micro-organisms** | **MTCC No.** | **Cilastatin +Imipenem** | **Meropenem** | **Etimicin** | **Drug of Invention with additive** |
| ***K.pneumoniae*** | 10031(ATCC) | 0.5 | 1 | 8 | 0.125 |
| ***P.vulgaris*** | 426 | 0.25 | 0.0625 | 4 | 0.015625 |
| ***P.aeruginosa*** | 1688 | 1 | 2 | 8 | 0.5 |
| ***E.coli*** | 1687 | 0.0625 | 0.0625 | 0.125 | 0.015625 |
| ***B.subtilis*** | 736 | 0.5 | 2 | 8 | 0.25 |
| ***E.cloacae*** | 509 | 0.25 | 0.125 | 2 | 0.0625 |
| ***S.aureus*** | 737 | 1 | 0.5 | 8 | 0.125 |
| ***C.braakii*** | 2690 | 0.0625 | 0.25 | 2 | 0.015625 |
| ***M.smegmatis*** | 995 | 0.125 | 0.25 | 2 | 0.015625 |
| ***A.baumanii*** | 1425 | 0.25 | 0.125 | 2 | 0.015625 |
| ***N.mucosa*** | 1722 | 0.25 | 0.125 | 1 | 0.015625 |
| ***MRSA*** | - | 0.5 | 0.5 | 4 | 0.0625 |

| | | | | | |
|---|---|---|---|---|---|
| * Drug of Invention- Meropenem + Etimicin + EDTA+PLP | | | | | |

**Table 2: Table showing that at 0.005mcg and 0.05mcg concentartions when meropenem, etimicin, ertapanem and their combinations without EDTA have either no zone of inhibition or the zone size is very very less compared to the FDC with EDTA and other additives indicating superiority of current invention.**

| **Culture:- *K. pneumoniae (10031 ATCC)*** | | | | | |
|---|---|---|---|---|---|
| S. No. | Drug Concentration | Zone of Inhibition | | | |
| | | **Meropenem** | **Etimicin** | **Mr+Eti** | **Mr+Eti+EDTA+X** |
| 1 | 0.005 | Nil | nil | nil | 20.33 |
| 2 | 0.05 | **14.36** | **nil** | **15.09** | **21.23** |
| 3 | 0.5 | **23.87** | **16.64** | **28.40** | **30.24** |

| | | **Ertapenem** | **Etimicin** | **Ert+Eti** | **Ert+Eti + EDTA+Y** |
|---|---|---|---|---|---|
| 1 | 0.005 | Nil | Nil | Nil | 18.67 |
| 2 | 0.05 | Nil | Nil | Nil | 20.98 |
| 3 | 0.5 | 15.90 | 16.50 | 19.26 | 23.57 |

| **Culture:- E. *coli (1687 MTCC)*** | | | | | |
|---|---|---|---|---|---|
| | | **Meropenem** | **Etimicin** | **Mr+Eti** | **Mr+Eti+EDTA+X** |
| 1 | 0.005 | 8.88 | Nil | 12.07 | 18.09 |
| 2 | 0.05 | 15.70 | 9.42 | 16.05 | 19.78 |
| 3 | 0.5 | 22.97 | 16.50 | 25.30 | 27.45 |

| | | **Ertapenem** | **Etimicin** | **Ert+Eti** | **Ert+Eti + EDTA+Y** |
|---|---|---|---|---|---|
| 1 | 0.005 | 10.10 | Nil | 10.70 | 17.79 |
| 2 | 0.05 | 15.49 | 9.31 | 16.21 | 22.03 |
| 3 | 0.5 | 21.45 | 15.70 | 22.44 | 24.67 |
| 4 | 1.0 | 23.39 | 17.57 | 24.23 | 26.99 |

| **Culture:- *C. braakii (2690 MTCC)*** | | | | | |
|---|---|---|---|---|---|
| | Drug Concentration (mcg) | **Meropenem** | **Etimicin** | **Mr+Eti** | **Mr+Eti+EDTA+X** |
| 1 | 0.005 | 9.35 | Nil | 13.10 | 19.67 |
| 2 | 0.05 | 23.35 | Nil | 23.90 | 29.88 |
| 3 | 0.5 | 28.60 | 18.70 | 32.42 | 37.07 |

| | Drug Concentration (mcg) | **Ertapenem** | **Etimicin** | **Ert+Eti** | **Ert+Eti + EDTA+Y** |
|---|---|---|---|---|---|
| 1 | 0.005 | 10.52 | Nil | 13.36 | 23.23 |
| 2 | 0.05 | 18.95 | Nil | 21.17 | 27.95 |
| 3 | 0.5 | 27.88 | 19.10 | 29.57 | 30.07 |

| **Culture:- *P. aeruginosa (1688 MTCC)*** | | | | | |
|---|---|---|---|---|---|
| S. No. | Drug Concentration (mcg) | **Zone of Inhibition** | | | |
| | | **Meropenem** | **Etimicin** | **Mr+Eti** | **Mr+Eti+EDTA** |
| 1 | 0.005 | Nil | Nil | Nil | 22.13 |
| 2 | 0.05 | 13.64 | Nil | 15.84 | 22.33 |
| 3 | 0.5 | 21.85 | 12.49 | 24.02 | 26.97 |
| | | | | | |

| | Drug Concentration (mcg) | **Ertapenem** | **Etimicin** | **Ert+Eti** | **Ert+Eti + EDTA+Y** |
|---|---|---|---|---|---|
| 1 | 0.005 | Nil | Nil | Nil | 22.80 |
| 2 | 0.05 | Nil | Nil | Nil | 23.10 |
| 3 | 0.5 | Nil | 12.60 | 12.73 | 24.31 |

| **Culture:- *S. aureus*** (737 MTCC) | | | | | |
|---|---|---|---|---|---|
| S. No. | Drug Concentration (mcg) | **Meropenem** | **Etimicin** | **Mr+Eti** | **Mr+Eti+EDTA+X** |
| 1 | 0.005 | 11.45 | Nil | 12.97 | 24.87 |
| 2 | 0.05 | 16.29 | 11.15 | 16.80 | 24.94 |
| 3 | 0.5 | 22.76 | 17.50 | 23.19 | 26.19 |

| | Drug Concentration (mcg) | **Ertapenem** | **Etimicin** | **Ert+Eti** | **Ert+Eti + EDTA+Y** |
|---|---|---|---|---|---|
| 1 | 0.005 | 9.44 | Nil | 11.16 | 25.10 |
| 2 | 0.05 | 14.55 | 10.12 | 14.75 | 24.35 |
| 3 | 0.5 | 21.33 | 16.65 | 21.80 | 25.86 |

| **Culture:- A. *baumannii* (1425 MTCC)** | | | | | |
|---|---|---|---|---|---|
| S. No. ' | Drug Concentration (mcg) | **Meropenem** | **Etimicin** | **Mr+Eti** | **Mr+Eti+ EDTA+X** |
| 1 | 0.005 | Nil | Nil | 12.40 | 18.57 |
| 2 | 0.05 | 14.50 | Nil | 17.35 | 18.89 |
| 3 | 0.5 | 19.81 | 13.5 | 20.37 | 24.26 |

| | | **Ertapenem** | **Etimicin** | **Ert+Eti** | **Ert+Eti + EDTA+Y** |
|---|---|---|---|---|---|
| 1 | 0.005 | Nil | Nil | Nil | 16.06 |
| 2 | 0.05 | 13.37 | 9.64 | 14.07 | 17.64 |
| 3 | 0.5 | 18.25 | 12.88 | 18.35 | 22.80 |

| **Culture:-** ***P. vulgaris** (426 MTCC)* | | | | | |
|---|---|---|---|---|---|
| S. No. | Drug Concentration (mcg) | **Meropenem** | **Etimicin** | **Mr+Eti** | **Mr+Eti+EDTA+X** |
| 1 | 0.005 | 10.84 | Nil | 11.60 | 20.81 |
| 2 | *0.05* | 18.01 | Nil | 18.77 | 21.58 |
| 3 | 0.5 | 21.32 | 15.19 | 21.87 | 24.56 |

| | | **Ertapenem** | **Etimicin** | **Ert+Eti** | **Ert+Eti + EDTA+Y** |
|---|---|---|---|---|---|
| 1 | 0.005 | 9.54 | Nil | 11.49 | 17.59 |
| 2 | 0.05 | 16.74 | Nil | 17.54 | 20.31 |
| 3 | 0.5 | 18.50 | 13.97 | 19.30 | 22.96 |

Where Xand Y are respective additive agents mixed in the formulation with water for injection. Thus, present invention shows synergy as per NCCLS (National Commmittee for Clinical Laboratory Standards) protocols and is better than individual drug.

**Table 3: Showing therapeutic drug concentration reduction by different ratios of combinations of Carbepenem and amoinoglycosides formulated in current invention.**

| | **Maimum Daily doses** | | | |
|---|---|---|---|---|
| | **Meropenem** | **Etimicin** | **Meropenem + Etimicin + Additives** | **%age Reduction in drug concentration** |
| **Adult** | 3-6gm | 400-800mg | 2160mg | 68.2 |
| **Pediatric** | 1.5-3gm | 200-400mg | 1080mg | 68.2 |
| **Neonates** | 750mg | 100-200mg | 135mg | 85.7 |

| | **Meropenm** | **Amikacin** | **Meropenem + Amikacin + Additives** | **%age Reduction in drug concentration** |
|---|---|---|---|---|
| **Adult** | 3-6gm | 1000mg | 2520mg | 64 |
| **Pediatric** | 1.5-3gm | 500mg | 1800mg | 48.5 |
| **Neonates** | 750mg | 375mg | 200mg | 74.6 |

| | **Meropenem** | **Gentamicin** | **Meropenem + Gentamicin + Additives** | **%age Reduction in drug concentration** |
|---|---|---|---|---|
| **Adult** | 3-6gm | 240mg | 2160mg | 65.3 |
| **Pediatric** | 1.5-3gm | 120mg | 1080mg | 65.3 |
| **Neonates** | 750mg | 37.5mg | 135mg | 82.8 |

| | **Imipenem** | **Gentamicin** | **Imipenem + Gentamicin + Additives** | **%age Reduction in drug concentration** |
|---|---|---|---|---|
| **Adult** | 1.5-3gm | 240mg | 1440mg | 55.5 |
| **Pediatric** | 750-1500mg | 120mg | 720mg | 55.5 |
| **Neonates** | 375-750mg | 37.5mg | 180mg | 77.1 |

| | **Ertapenem** | **Etimicin** | **Ertapenem + Etimicin + Additives** | **%age Reduction in drug concentration** |
|---|---|---|---|---|
| **Adult** | 1-2gm | 400-800mg | 900mg | 67.8 |
| **Pediatric** | 500-1000mg | 200-400mg | 480mg | 48 |
| **Neonates** | 250-500mg | 100-200mg | 160mg | 77.1 |

| | **Panipenem** | **Etimicin** | **Panipenem + Etimicin** + **Additives** | **%age Reduction in drug concentration** |
|---|---|---|---|---|
| **Adult** | 1-2gm | 400-800mg | 900mg | 67.8 |
| **Pediatric** | 500-1000mg | 200-400mg | 240mg | 82.8 |
| **Neonates** | 200mg | 100-200mg | 160mg | 6C |

| | **Ertapenem** | **Gentamicin** | **Ertapenem + Gentamicin + Additives** | **%age Reduction in drug concentration** |
|---|---|---|---|---|
| **Adult** | 1-2gm | 240mg | 900mg | 59.8 |
| **Pediatric** | 500-1000mg | 120mg | 240mg | 78.5 |
| **Neonates** | 250-500mg | 37.5mg | 120mg | 77.6 |

### BRIEF DESCRIPTION OF DRAWINGS:

Fig. 1: Graph showing decrease of SOD activity in renal tissue after administration of all the antibiotics(Meropenem, Amikacin, Tobramycin, Etimicin) except Drug of Invention
Fig. 2: Graph showing decrease of Catalase activity in renal tissue after administration of all the antibiotics(Meropenem, Amikacin, Tobramycin, Etimicin) except Drug of Invention
Fig. 3: Graph showing increase of MDA activity in renal tissue after administration of all the antibiotics(Meropenem, Amikacin, Tobramycin, Etimicin) except Drug of Invention
Fig. 4: Graph showing decrease of Glutathione reductase activity in renal tissue after administration of all the antibiotics(Meropenem, Amikacin, Tobramycin, Etimicin) except Drug of Invention
Fig. 5 : Creatnine level in renal tissue of Mus musculus mice
Fig. 6: Proof of overcoming drug resistance in 3 bacterial strains resistant to penems

### DETAILED DESCRIPTION OF DRAWINGS

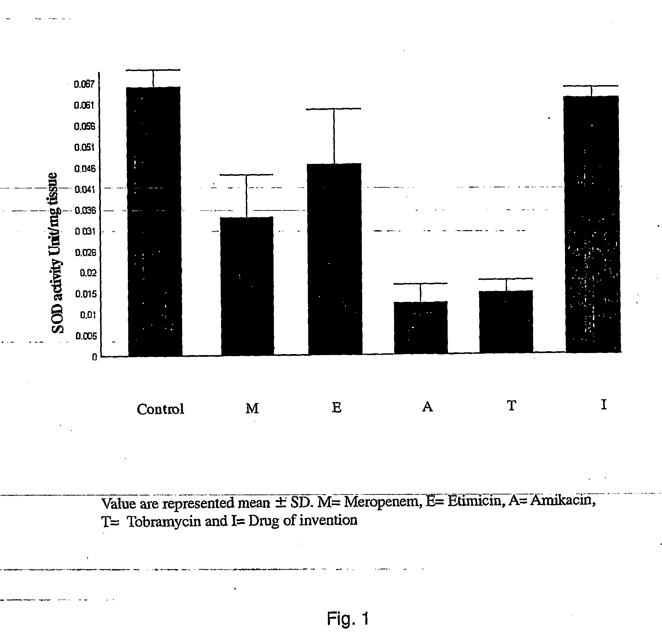

Fig. 1: The level of SOD (Superoxide dismutase)was significantly enhanced in (I) drug of invention group and reached near to normal level which shows that drug of invention inhibits toxicity and is safe parallel to control group.

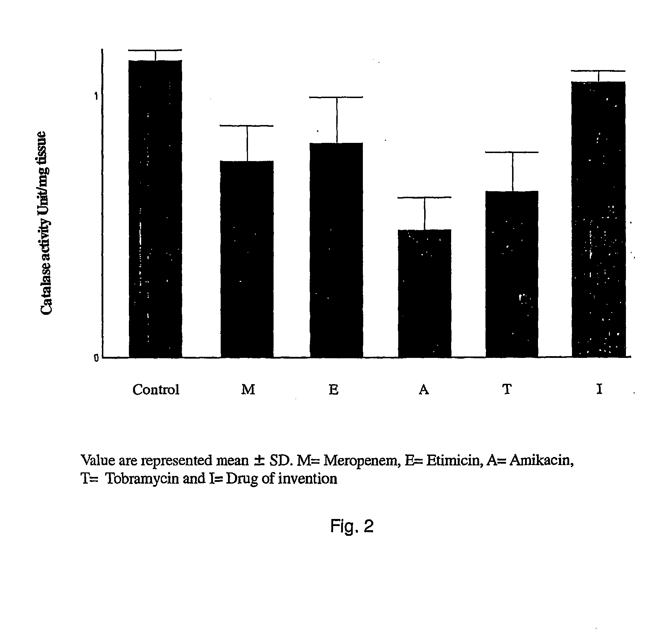

Fig. 2 : The activity of catalase was found to be statistically significantly increased in (I) drug of invention and reached almost near to normal level, which is related to reduction in oxidative stress generated by other antibiotic administration.

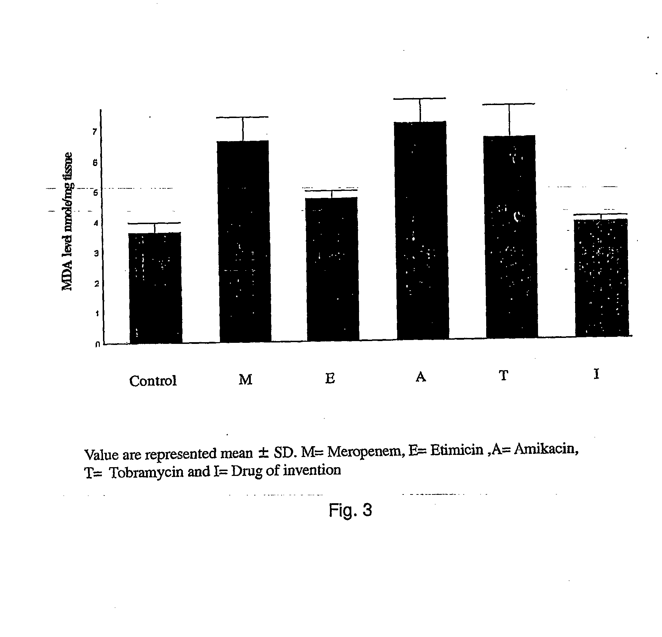

Fig. 3 : Drug of invention shows lesser toxicity as compared to Meropenem, Etimicin, amikacin and Tobramycin.

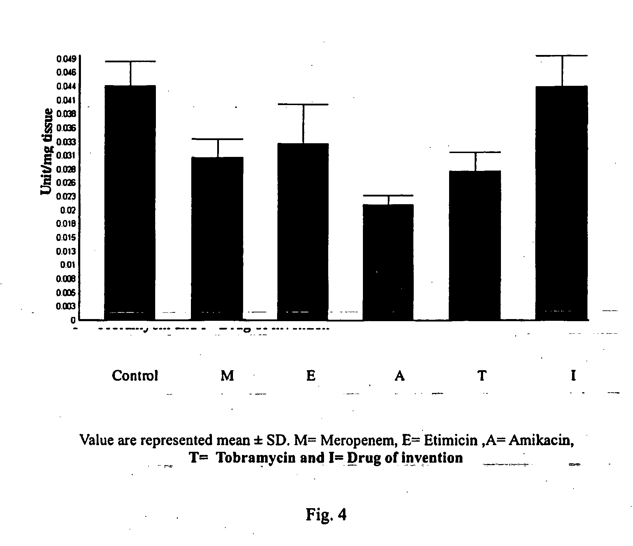

Fig. 4 : Drug of invention is safe and does not cause kidney toxicity.

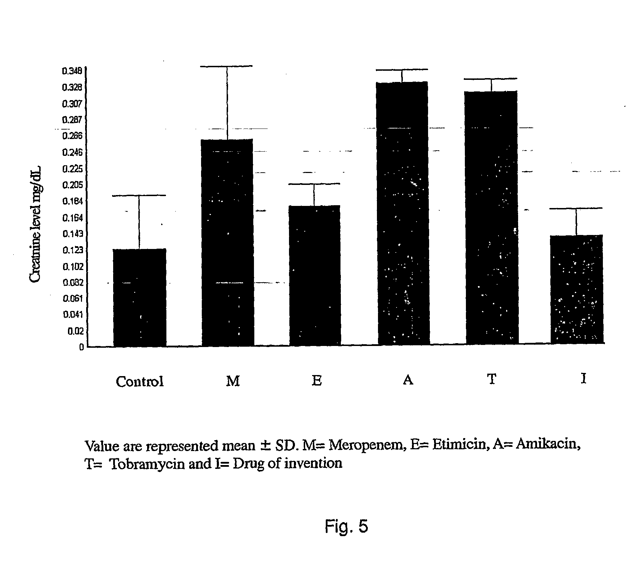

Fig. 5: The level of creatinine was found to be significantly decreased in Etimicin and product of invention treated group and reached almost near to normal level stating that drug does not cause kidney toxicity after repeated administration to mice for 7 days consecutively.
   Over all conclusion of present finding revealed that our product of invention (I) possess antioxidative and free radical scavenging potential that contribute in improving the efficacy and safety profiles. It was also concluded that Etimicin is also effective antibiotic against oxidative stress and helpful for maintenance of antioxidant levels in renal tissue.

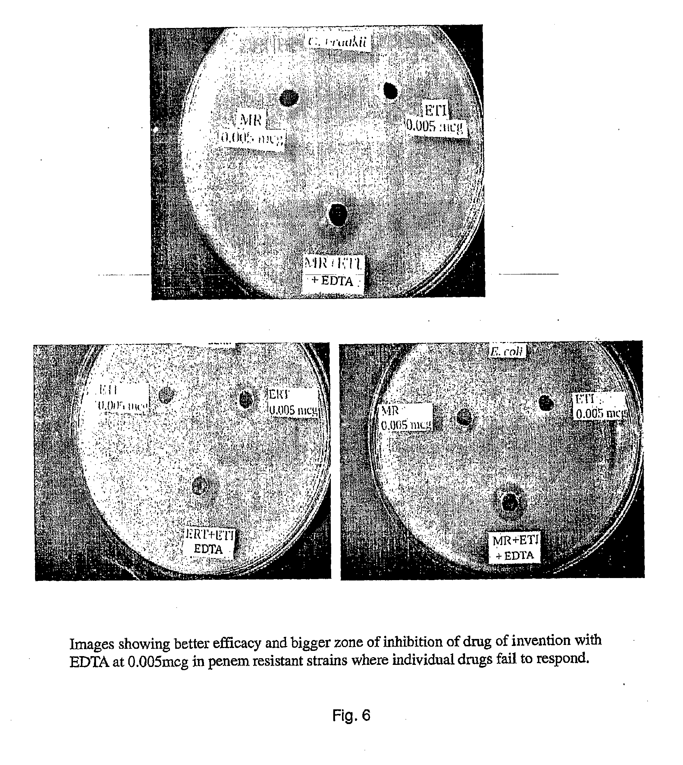

Fig. 6: Images showing better efficacy and bigger zone of inhibition of drug of invention with EDTA at 0.005mcg in penem resistant strains of *C.brakki, E.coli and A.baumanii* where individual drugs fail to respond

## Claims

1. A pharmaceutical formulation comprising two different groups of antibiotics as active constituents present as synergistic fixed dose combination in parenteral dosage form with bactericidal efficacy at a very low concentration wherein:
**(a)** first antibiotics is a carbapenem selected from a group of meropenem, ertapenem, doripenem, imipenem, panipenem or a pharmaceutically acceptable salt thereof,
**(b)** second antibiotic is an aminoglycoside which is etimicin or a pharmaceutically acceptable salt thereof,
**(c)** both antibiotics are present in weight ratios of 6:1 to 13:1 along with
**(d)** one or more additives selected from a group of synthetic/ natural amino acids/ vitamins/ stabilizers/ polymers/ antioxidants/ micronutrients where the weight ratio of active constituents : additives is 1: 0.0001 to 1: 0.75 thereby enabling the composition and formulation made thereof to overcome carbapenem and aminoglycoside resistance and to lower drug and disease induced toxicity,
**(e)** said additives are either added directly in the composition at the time of formulation or could also be added to a solvent used for reconstitution of said formulation,
**(f)** the composition thus formed has improved efficacy with dose lower than the individually established therapeutic drug concentrations of active constituents,
**(g)** the said composition is safe and is present as single unit premix dry powder injection formulation to be reconstituted with solvent which is preferably water for injection or alternatively contains one or more of the additive agents.

2. The formulation as disclosed in claim 1 wherein:
**(a)** the composition is a single unit, packed in a sealed air tight pharmaceutically acceptable container, present as dry powder for reconstitution, lyophilized powder, as a dose concentrate in the form of drug particles, powders, granules, nano-particles and micorspheres
**(b)the** said composition is suitably protected from moisture, light and degradation with the help of vacuum or inert gas micro atmosphere or in pressurized carbon dioxide.

3. The formulation of claim 1 to 2 for use for the treatment of patients suffering from or susceptible to mixed multi bacterial fatal infections.

4. A process of preparing a composition of claim 1 - 2 comprising the step of:
**(a)** sterile filling / blending the antibiotic ingredients or pharmaceutically acceptable salts thereof,
**(b)** sterile adding / blending the additives
**(c)** continuing said sterile adding / filling / blending for a period ranging from about 1 hour to about 6 hours,
**(d)** proportioning the sterile fill / blend, and
**(e)** capping aseptically with pre-post inert gassing.

## Patentansprüche

1. Pharmazeutische Formulierung, die zwei verschiedene Gruppen Antibiotika als aktive Bestandteile umfasst, die als synergetische Festdosiskombination in parenteraler Dosierungsform mit bakterizider Wirksamkeit in einer sehr geringen Konzentration vorliegen, wobei:
**(a)** es sich bei dem ersten Antibiotikum um ein Carbapenem handelt, das aus einer Gruppe von Meropenem, Ertapenem, Doripenem, Imipenem oder einem pharmazeutisch annehmbaren Salz von diesen ausgewählt ist,
**(b)** es sich bei dem zweiten Antibiotikum um ein Aminoglykosid handelt, das Etimicin oder ein pharmazeutisch annehmbares Salz von diesem ist,
**(c)** beide Antibiotika in Gewichtsverhältnissen von 6:1 bis 13:1 vorliegen, zusammen mit
**(d)** einem oder mehreren Additiv/en, das/die aus einer Gruppe aus synthetischen/natürlichen Aminosäuren/Vitaminen/Stabilisatoren/Polymeren/Anti-oxidantien/Mikronährstoffen ausgewählt ist/sind, wobei die Gewichtsverhältnisse von Wirkbestandteilen : Additiven 1 : 0,0001 bis 1 : 0,75 betragen, wodurch es der daraus hergestellten Zusammensetzung und Formulierung ermöglicht wird, eine Carbapenem-und Aminoglykosidresistenz zu überwinden und medikament- und krankheitsinduzierte Toxizität zu senken,
**(e)** die Additive entweder direkt der Zusammensetzung zum Formulierungszeitpunkt zugesetzt werden oder auch einem Lösungsmittel zugesetzt werden könnten, das zur Rekonstitution der Formulierung verwendet wird,
**(f)** die so gebildete Zusammensetzung eine verbesserte Wirksamkeit bei geringerer Dosis als die individuell ermittelten therapeutischen Medikamentenkonzentrationen an aktiven Bestandteilen hat,
**(g)** die Zusammensetzung sicher ist und als Einzeleinheit-Vormischtrockenpulver-injektionsformulierung vorliegt, um mit Lösungsmittel rekonstituiert zu werden, bei dem es sich vorzugsweise um Wasser für Injektionszwecke handelt oder alternativ einen oder mehrere der Zusatzstoffe enthält.

2. Formulierung nach Anspruch 1, wobei:
**(a)** die Zusammensetzung eine in einem versiegelten, luftdichten, pharmazeutisch annehmbaren Behälter verpackte Einzeleinheit ist, als Trockenpulver zur Rekonstitution, lyophilisiertes Pulver, als Dosiskonzentrat in Form von Medikamentenpartikeln, -pulver, -granulat, -nanopartikeln und -mikrokügelchen vorliegt,
**(b)** die Zusammensetzung vor Feuchtigkeit, Licht und Abbau mit Hilfe einer Vakuum- oder Inertgasmikroatmosphäre oder in Druckkohlendioxid angemessen geschützt ist.

3. Formulierung nach Anspruch 1 oder 2 zur Verwendung für die Behandlung von Patienten, die an lebensbedrohlichen multibakteriellen Mischinfektionen leiden oder dafür anfällig sind.

4. Verfahren zum Herstellen einer Zusammensetzung nach Anspruch 1 - 2, folgende Schritte umfassend:
**(a)** steriles Zufüllen/Vermischen der antibiotischen Inhaltsstoffe oder derer pharmazeutisch annehmbaren Salze,
**(b)** steriles Zusetzen/Vermischen der Additive,
**(c)** Fortsetzen des sterilen Zusetzens/Zufüllens/Vermischens über einen Zeitraum, der von ca. 1 Stunde bis ca. 6 Stunden reicht,
**(d)** Portionieren der sterilen Füllung/Mischung, und
**(e)** aseptisches Verschließen mit einem Deckel bei vorheriger/nachheriger Inertbegasung.

## Revendications

1. Formulation pharmaceutique comprenant deux groupes différents d'antibiotiques comme constituants actifs présents comme combinaison à dose fixe synergiste sous forme posologique parentérale à efficacité bactéricide à très faible concentration, dans laquelle :
**(a)** un premier antibiotique est un carbapénème choisi dans un groupe de méropénème, ertapénème, doripénème, imipénème, panipénème ou un sel pharmaceutiquement acceptable de ceux-ci,
**(b)** un deuxième antibiotique est un aminoglycoside qui est de l'étimicine ou un sel pharmaceutiquement acceptable de celle-ci,
**(c)** les deux antibiotiques sont présents dans des rapports de poids de 6:1 à 13:1 ainsi que
**(d)** un ou plusieurs additifs choisis dans un groupe d'acides aminés synthétiques / naturels / vitamines / stabilisateurs / polymères / antioxydants / micronutriments, le rapport de poids constituants actifs : additifs étant de 1:0,0001 à 1:0,75, permettant ainsi à la composition et à la formulation élaborée à partir de celle-ci de surmonter la résistance aux carbapénèmes et aux aminoglycosides et d'abaisser la toxicité induite par les médicaments et la maladie,
**(e)** lesdits additifs sont soit ajoutés directement dans la composition au moment de la formulation ou pourraient aussi être ajoutés à un solvant utilisés pour la reconstitution de ladite formulation,
**(f)** la composition ainsi formée a une efficacité accrue à dose inférieure aux concentrations médicamenteuses thérapeutiques établies individuellement de constituants actifs,
**(g)** ladite composition est sûre et est présente comme formulation d'injection en poudre sèche prémélangée en unité unique à reconstituer avec du solvant qui est de préférence de l'eau pour injection ou en variante contient un ou plusieurs des agents additifs.

2. La formulation telle qu'exposée dans la revendication 1, dans laquelle :
**(a)** la composition est une unité unique, emballée dans un récipient pharmaceutiquement acceptable hermétique étanche à l'air, présente comme poudre sèche pour reconstitution, poudre lyophilisée, comme concentré de dose sous la forme de particules médicamenteuses, poudres, granules, nanoparticules et microsphères,
**(b)** ladite composition est adéquatement protégée contre l'humidité, la lumière et la dégradation à l'aide de vide ou de microatmosphère de gaz inerte ou dans du dioxyde de carbone sous pression.

3. La formulation de la revendication 1 à 2 destinée à être utilisée pour le traitement de patients souffrant d'infections multibactériennes mixtes mortelles ou sujets à celles-ci.

4. Procédé d'élaboration d'une composition de la revendication 1 - 2 comprenant les étapes de :
**(a)** remplissage / mélange stérile des ingrédients antibiotiques ou sels pharmaceutiquement acceptables de ceux-ci,
**(b)** ajout / mélange stérile des additifs,
**(c)** poursuite dudit ajout / remplissage / mélange stérile pendant une période allant d'environ 1 heure à environ 6 heures,
**(d)** dosage dudit remplissage / mélange stérile, et
**(e)** obturation aseptisée avec gazage préalable et consécutif au gaz inerte.
